# EUROPÄISCHE PATENTSCHRIFT

(11) **EP 0 603 671 B1**
(45) Veröffentlichungstag und Bekanntmachung des Hinweises auf die Patenterteilung: **11.11.1998**
(21) Anmeldenummer: 93119912.9
(22) Anmeldetag: 10.12.1993
(51) Int. Cl.: C07K 1/00, C07K 1/22, C07K 14/745, G01N 33/86, A61K 38/36

(54) **Verfahren zur Reaktivierung von gereinigten Membranproteinen**
Process of reactivating purified membrane proteins
Méthode de réactivation de protéines membranaires purifiées

(30) Priorität: 23.12.1992 DE 4243729
(43) Veröffentlichungstag der Anmeldung: 29.06.1994
(73) Patentinhaber: Dade Behring Marburg GmbH, 35001 Marburg (DE)
(72) Erfinder: Fickenscher, Karl, D-35041 Marburg (DE); Zander, Norbert F., D-35041 Marburg (DE)

(56) Entgegenhaltungen:
- EP-A- 0 355 845
- EP-A- 0 433 225
- WO-A-92/08479
- US-A- 5 149 529
- DATABASE WPI Section Ch, Week 9035, Derwent Publications Ltd., London, GB; Class B04, AN 90-266194 & JP-A-2 188 532 (OTSUKA PHARM KK) 24. Juli 1990

## Beschreibung

Die Erfindung betrifft ein Verfahren zur Reaktivierung von gereinigten Membranproteinen.

Membranproteine (z. B. Rezeptoren) bestehen aus einer oder mehreren Transmembrandomänen sowie intra- und extrazellulären Domänen. Die Aktivität solcher Proteine wird häufig nach Integration des aufgereinigten Proteins in eine künstliche Membran gemessen.

Als Beispiel kann der Gewebsfaktor (Gewebs-Thromboplastin) dienen. Dieser Rezeptor für den Faktor VII des Blutgerinnungssystems besteht aus Apoprotein sowie Lipiden (Pitlick, F. A. et al.(1970), Biochemistry 9, 5109-5113). Das Apoprotein ist ein glykosyliertes Polypeptid von 263 Aminosäuren. Nahe dem carboxyterminalen Ende besitzt es eine hydrophobe Sequenz von 23 Aminosäuren, mit der es in der Membran verankert ist. Der intrazelluläre Anteil besteht aus 21 Aminosäuren (Fisher et al.(1987), Thromb. Res. 48, 89 - 99); Morrissey et al.(1987), Cell 50, 129 - 135). In vivo kommt der Gewebsfaktor als integrales Membranprotein von Zellen vor, die nicht in direktem Kontakt mit Blut stehen. Seine physiologische Funktion besteht darin, als Zelloberflächenrezeptor bei Kontakt mit Blut bzw. Plasma den plasmatischen Gerinnungsfaktor VII zu binden und zu aktivieren. Dieser Komplex besitzt Serinprotease-Aktivität und ist seinerseits in der Lage, die Faktoren IX und X zu aktivieren und damit die Gerinnung auszulösen.

Bei der Isolation von Gewebsfaktor können zwei Methoden unterschieden werden. Bei der einen wird der aktive Gewebsfaktor teilweise gereinigt, indem geeignete Gewebe aufgeschlossen und die entsprechende Membranfraktion isoliert wird. Verwendung findet dieses Material in erster Linie bei der Herstellung von diagnostisch eingesetzten Reagenzien zur Überprüfung der plasmatischen Blutgerinnung. Da das Membranprotein als ganzes (d. h. inklusive gebundener Lipidmoleküle) isoliert wird, ist eine Reaktivierung des Apoproteins nicht notwendig.

Bei den anderen Verfahren wird das isolierte Apoprotein gewonnen. Da es fast keine Aktivität mehr besitzt (Broze, G. J. et al.(1985), J.Biol.Chem. 260, 10917-10920), muß es reaktiviert werden. Hierzu ist es erneut in eine LipidMembran zu integrieren (Pitlick et al. (1970), Biochemistry 9, 5105-5113); Bach et al. (1981), J.Biol.Chem. 256, 8234-8331). Unumgänglich ist dieses Verfahren für Protein, das auf rekombinantem Wege gewonnen wird, da die mikrobiologischen Organismen, die zur Produktion eingesetzt werden, in der Regel nicht in der Lage sind, ein ausreichend aktives Membranprotein zu liefern. Grundsätzlich gelten diese Überlegungen auch für Proteine, die durch Teil- oder Totalsynthese in vitro hergestellt werden können.

Zum Wiedereinbau aufgereinigter Membranproteine in eine Lipidmembran sind eine Reihe von Verfahren bekannt. Üblicherweise werden dazu Phospholipide mit einem Detergenz, z. B. Deoxycholat, in eine wäßrige Lösung gebracht. Diese wird mit dem gereinigten Membranprotein gemischt. Anschließend wird das Detergenz entfernt, zum Beispiel durch Dialyse. Beim Mischen des Apoproteins mit der Phospholipidlösung bzw. bei der Entfernung des Detergenzes erfolgt der Einbau des Proteins in die sich bildenden Membranvesikel (Pitlick, s.o.). Deoxycholat ist das bevorzugte Detergenz, da es durch Dialyse entfernt werden kann. Prinzipiell sind andere Detergenzien jedoch auch einsetzbar, wenn man sie wieder aus dem Ansatz entfernen kann.

So beschrieben Wijngaards et al. ((1977) Biochim. Biophys. Acta 488, 161 - 171) ein Verfahren zur Relipidisierung von Gewebsfaktor unter Verwendung von Natriumtaurocholat als Detergenz. Sie finden ein pH-Optimum bei pH 4.0, sind aber ebenfalls gezwungen, das Detergenz nach erfolgter Relipidisierung aus dem Ansatz zu entfernen.

Ein grundsätzlicher Nachteil bei der Verwendung von Detergenzien ist, daß diese Hilfsmittel unbedingt wieder aus dem Ansatz entfernt werden müssen. Sie vermitteln zwar den Einbau vom Protein in eine Membran, stören jedoch stark die Aktivität des Membran-Protein-Komplexes, da sie selbst ebenfalls in die Lipidvesikel integriert werden. Zur Entfernung des Detergenzes wird der Ansatz in der Regel gegen ein großes Volumen dialysiert. Das ist zeitaufwendig, umständlich und führt zu Ausbeute- und Aktivitätsverlusten. Ferner ist die Entfernung der Detergenzien auf diese Weise nicht vollständig möglich.

Der Erfindung lag die Aufgabe zugrunde, ein einfaches Verfahren zur Verfügung zu stellen, mit dessen Hilfe gereinigte Membranproteine ohne die Verwendung von Detergentien so reaktiviert werden können, daß sie funktional den Proteinen in physiologischer Umgebung entsprechen. Diese Reaktivierung besteht im Einbau des entsprechenden Membranproteins in geeignete Lipidmizellen (Relipidisierung). Im Falle des gereinigten Gewebsfaktor-Apoproteins kann nur so die natürliche gerinnungsphysiologische Aktivität wiederhergestellt werden.

Überraschenderweise wurde gefunden, daß eine Relipidisierung ohne Zuhilfenahme von Detergentien durch Ansäuern und/oder Erhitzen einer Protein-/Lipidmischung erreicht werden kann.

Grundsätzlich kann das erfindungsgemäße Verfahren auch auf Mischungen gereinigter Proteine angewendet werden.

Es wurde gefunden, daß die Relipidisierung erreicht werden kann, indem Protein und Phospholipide bei ausreichend niedrigen pH-Werten gemischt werden. Dabei müssen die Phospholipide nicht mit Hilfe eines Detergenzes gelöst werden, sondern es genügt, sie in einer wäßrigen Lösung zu emulgieren. Unmittelbar nach homogener Durchmischung des Ansatzes kann bereits der pH wieder auf den gewünschten Wert eingestellt werden. Geeignete pH-Bereiche liegen zwischen pH 1 und 5, bevorzugterweise zwischen pH 2 bis 4, besonders bevorzugterweise bei einem pH von etwa 3. Als Phospholipid kann dem Fachmann an sich bekanntes Material pflanzlichen oder tierischen Ursprungs als natürliche Mischung unterschiedlicher Komponenten verwendet werden. Ebenfalls einsetzbar sind dem Fachmann bekannte definierte Reinsubstanzen oder Mischungen davon. Bevorzugterweise wird in dem erfindungsgemäßen Verfahren mit pflanzlichen Phospholipidgemischen gearbeitet.

Die Relipidisierung kann mit einem gelösten oder einem an eine Affinitätssäule (z. B. eine Immunadsorptionssäule mit einem polyklonalen oder monoklonalen Antikörper) gebundenen Membranprotein durchgeführt werden. Grundsätzlich kann ein Membranprotein humanen, pflanzlichen, tierischen, mikrobiellen oder rekombinanten Ursprungs verwendet werden; ebenfalls möglich ist die Verwendung einer Mutante eines natürlich vorkommenden Proteins. Bevorzugt ist die Verwendung eines gelösten Membranproteins in einer Konzentration von 0 bis zu 50 mg/ml. In diesem Fall wird zunächst eine wäßrige Emulsion von Phospholipiden von 0 bis zu 50 mg/ml mit Puffer bei saurem pH gemischt. Zu dieser sauren Emulsion wird anschließend aufgereinigtes Membranprotein gegeben und gemischt. Nach einer Inkubationszeit von etwa 1 bis 10 min, bevorzugterweise von 2 bis 6 min, wird der Ansatz durch Zugabe von Puffer neutralisiert.

Die der Erfindung zugrundeliegende Aufgabe kann auch in einer zweiten Ausführungsform zur Integration von Membranproteinen in eine Lipidmembran gelöst werden, nämlich durch das Erhitzen eines Proteins in Gegenwart von Phospholipiden. Wie beim Verfahren durch Ansäuern ist es unnötig, die Lipide mit Hilfe von Detergenzien zu lösen. Als Phospholipide können die bereits oben genannten Materialien verwendet werden.

Auch für die einzusetzenden Membranproteine sowie die bevorzugten Konzentrationsbereiche für Protein und Phospholipide gilt das oben Gesagte.

Bevorzugt ist eine Erhitzung auf 80 bis 95 °C für 1 bis 10 min, besonders bevorzugterweise für etwa 4 bis 6 min. Anschließend wird der Ansatz innerhalb von 1 bis 10 min, bevorzugterweise für etwa 4 bis 6 min auf Raumtemperatur gekühlt und anschließend mit Puffer versetzt.

Nach der Relipidisierung liegt das Membranprotein in aktiver Form eingebaut in eine Lipidmembran vor. Es kann dann zur weiteren Verwendung gegebenenfalls mit Zusätzen versehen und konfektioniert werden. Wird Gewebsfaktor-Apoprotein mit einem der erfindungsgemäßen Verfahren relipidisiert, ist eine Verwendung als Therapeutikum oder als Diagnostikum möglich. Im zweiten Fall kann der relipidisierte Gewebsfaktor insbesondere zu einem Reagenz zur Bestimmung der Prothrombinzeit zum Zwecke der Überprüfung der plasmatischen Blutgerinnung verarbeitet werden.

Am Beispiel des Gewebsfaktor-Apoproteins soll die Erfindung im folgenden näher erläutert werden.

### Beispiel 1:

### Relipidisierung eines rekombinant gewonnenen und gereinigten humanen Gewebsthromboplastin-Apoproteins durch Ansäuern

Menschliches Gewebsfaktor-Apoprotein wurde in E. coli exprimiert. Das Protein wurde aus dem E. coli-Extrakt über eine Immunabsorptionssäule aufgereinigt und auf 10 µg/ml verdünnt.

Zu einem Relipidisierungsansatz wurden zusammengegeben:
- 50 µl: Phospholipidsuspension (0.5 % w/v Phospholipon 25 P, in Aqua dest., Fa. Nattermann, Germany)
- 50 µl: 0.1 M Glyzin, auf pH 2.5 eingestellt mit HCl

Nach Mischen wurde 5 min bei Raumtemperatur inkubiert und anschließend
- 100 µl: Gewebsfaktor-Apoprotein
zugegeben. Nach erneutem Mischen wurde 1 min bei Raumtemperatur inkubiert und
- 800 µl: 50 mM (N-{2-Hydroxyethyl}piperazine-N'-{2-Ethansulfonsäure)), 5 g/l Glyzin, 13 mM CaCl₂, 0.1 % NaN₃, pH 7.5
zugegeben.

Die Bestimmung der Prothrombinzeit erfolgte an einem Koagulometer nach Schnitger und Gross (0.1 ml humaner Normalplasmapool + 0.2 ml relipidisierter Gewebsfaktor) und ergab 11.0 s Gerinnungszeit.

### Beispiel 2:

### pH-Abhängigkeit der Relipidisierung

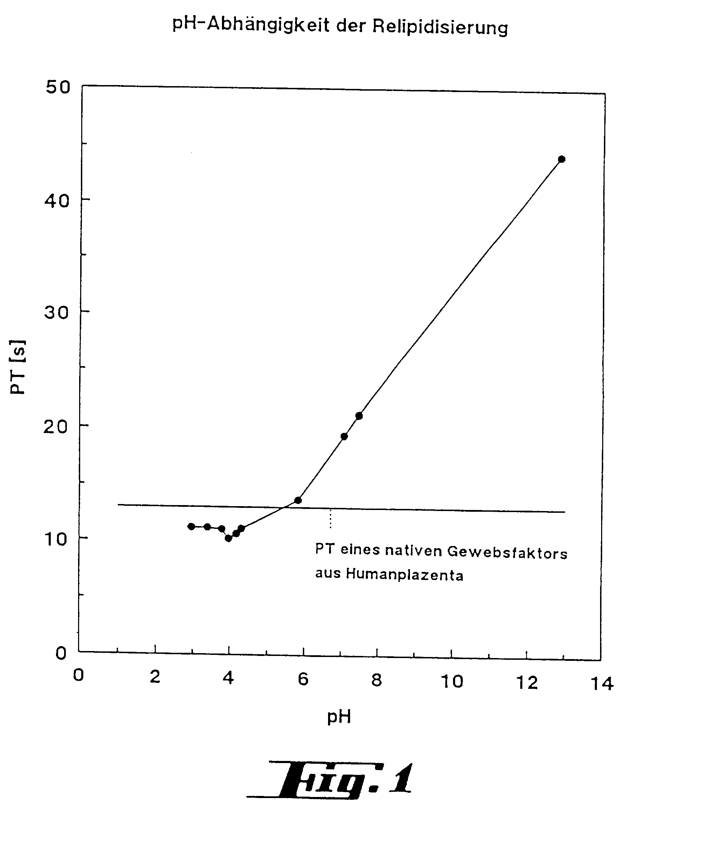

Die Relipidisierung des Gewebsfaktors erfolgte wie in Beispiel 1 beschrieben. Es wurden jedoch Glyzinpuffer mit verschiedenen pH-Werten eingesetzt (pH 1.5 bis 13.5). Nach Mischen der Phospholiponsuspension mit dem Gewebsfaktor wurde der pH gemessen. Figur 1 stellt den Zusammenhang zwischen dem gemessenen pH-Wert im Relipidisierungsansatz und der erhaltenen Prothrombinzeit dar. Es ergibt sich ein pH-Optimum zwischen pH 3 und pH 5. In diesem Bereich sind die gemessenen Prothrombinzeiten vergleichbar mit Zeiten, die mit derselben Menge nativem, aus Gewebe isoliertem, Gewebsfaktor gemessen werden. Niedrigere pH-Werte waren bei der Zusammensetzung der gewählten Puffer im Relipidisierungsansatz nicht erreichbar, sollten aber ebenfalls geeignet sein.

### Beispiel 3:

### Zeitabhängigkeit der Relipidisierung durch Ansäuern

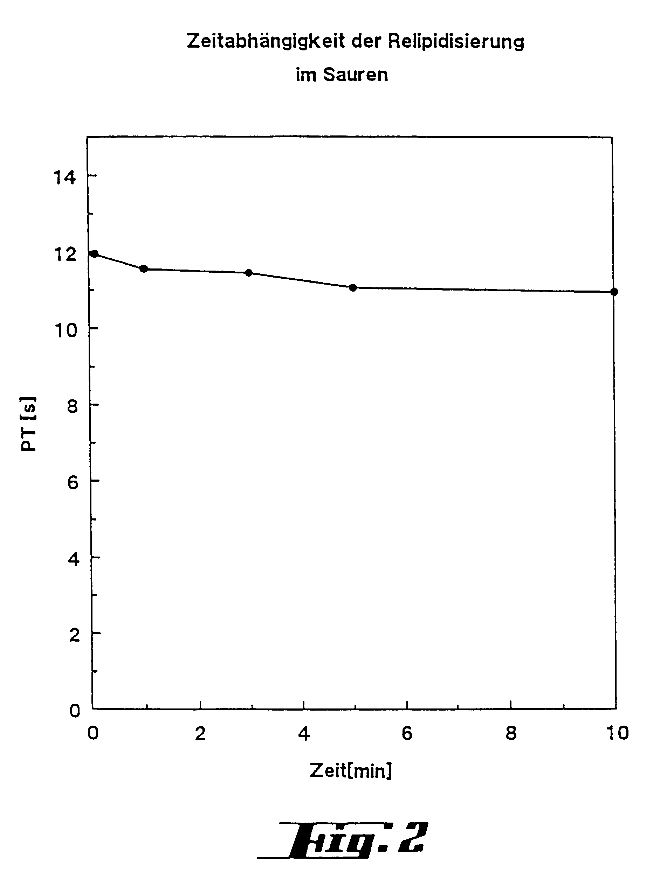

Die Relipidisierung erfolgte wie in Beispiel 1 beschrieben. Das Neutralisieren erfolgte nach verschiedenen Zeitintervallen von 10 s bis 10 min nach Mischen von Lipid und Protein. Anschließend wurde die Prothrombinzeit eines humanen Normalplasmapools koagulometrisch bestimmt. Figur 2 zeigt den Zusammenhang zwischen der Länge der Inkubation bei saurem pH und den gemessenen Prothrombinzeiten. Die Prothrombinzeit ist von der Länge der Inkubation bei saurem pH nur schwach abhängig; eine Inkubation von 5 bis 10 min Länge liefert die kürzeste Prothrombinzeit.

### Beispiel 4:

### Relipidisierung durch Erwärmen

Zu einem Relipidisierungsansatz wurden zusammengegeben:
- 100 µl: Gewebsfaktor-Apoprotein (10 µg/ml)
- 100 µl: 0.5 % (w/v) Phospholipon 25 P in Aqua dest.

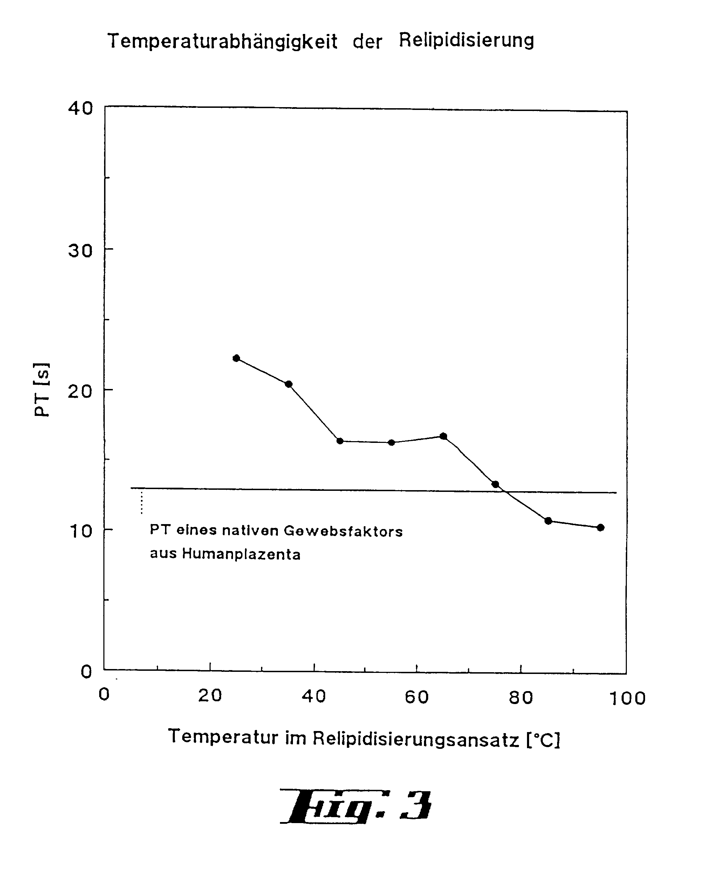

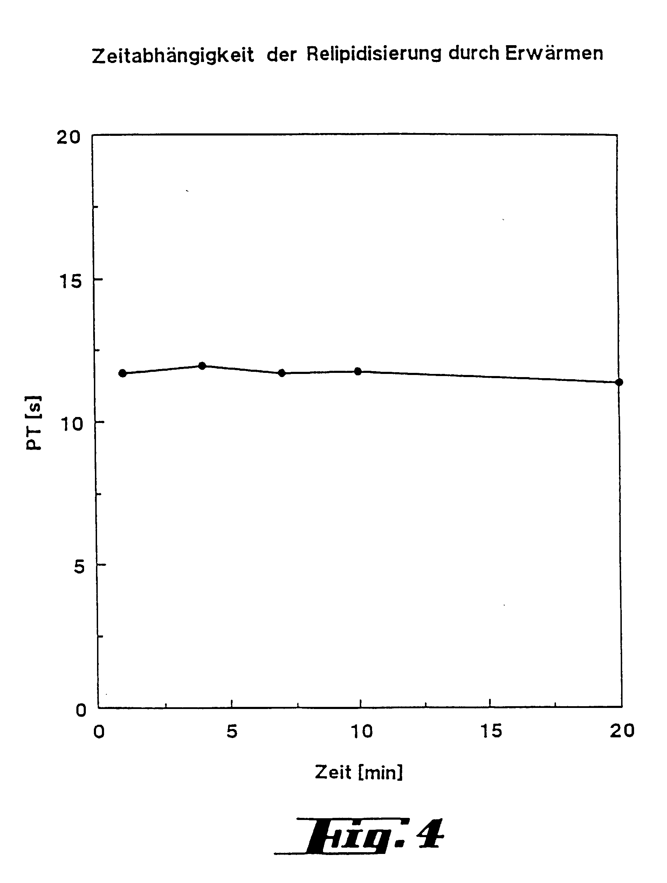

Der Ansatz wurde 5 min lang einer Temperatur zwischen 25 °C und 95 °C ausgesetzt. Anschließend wurde innerhalb von 5 Minuten auf Raumtemperatur abgekühlt und
- 800 µl: 50 mM (N-{2-Hydroxyethyl}piperazine-N'⁻{2-Ethan sulfonsäure}), 5 g/l Glyzin, 13 mM CaCl₂, 0.1 % NaN₃, pH 7.5
wurden zugegeben. Die Prothrombinzeit eines humanen Normalplasmapools wurde an einem Koagulometer nach Schnitger und Groß bestimmt. Figur 3 zeigt den Zusammenhang zwischen der Temperatur beim Hitzeschritt und der gemessenen Prothrombinzeit. Die kürzeste Zeit ergibt sich bei einem Hitzeschritt von 95 °C. Temperaturen über 80 °C liefern kürzere Prothrombinzeiten als die mit einer entsprechenden Menge nativen Gewebsfaktors gemessenen. Figur 4 zeigt den Zusammenhang zwischen der Länge eines Hitzeschritts bei 95 °C und der gemessenen Prothrombinzeit eines humanen Normalplasmapools. Die Relipidisierung läßt sich durch einen Hitzeschritt von nur 1 min Dauer erreichen.

### Beispiel 5:

### Qualität der Reagenzien: Faktor VII-Empfindlichkeit

Ein humaner Normalplasmapool wurde mit einem Faktor VII-Mangelplasma (Behringwerke AG, Marburg, Germany) verdünnt. Es wurden Faktor VII-Konzentrationen zwischen 100 % und 10 % (bezogen auf den humanen Normalplasmapool) eingesetzt.

Die Prothrombinzeiten der Proben wurden anschließend an einem Koagulometer nach Schnitger und Groß bestimmt. Die gemessenen Zeiten wurden ins Verhältnis zu der Prothrombinzeit des humanen Normalplasmapools eingesetzt.

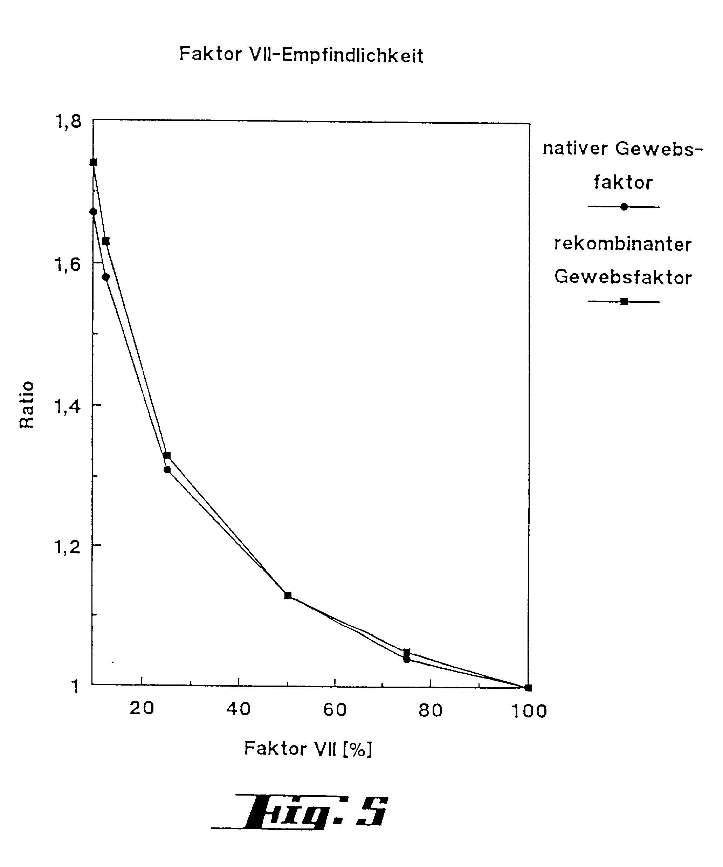

Figur 5 zeigt die relativen Prothrombinzeiten [Ratio = (PT) / (PT_{100 % Faktor VII})] in Abhängigkeit von der Faktor VII-Konzentration der Probe. Es wurden zwei Gewebsfaktor-Reagenzien verwendet:
a) Nativer Gewebsfaktor, isoliert aus menschlicher Plazenta (Thromborel S, Behringwerke AG)
b) Reagenz, ausgehend von rekombinantem Gewebsfaktor-Apoprotein, das nach einem erfindungsgemäßen Verfahren (wie in Beispiel 1) hergestellt wurde.

Der Normalbereich der Faktor VII-Konzentration in einer menschlichen Population (95-Perzentil) wird durch Prothrombin-Ratios von 1.00 ± 0.20 erfaßt. Plasmen, die eine außerhalb dieses Bereiches liegende Prothrombin-Ratio aufweisen, werden als pathologisch erkannt. Die dieser Empfindlichkeitsgrenze entsprechende Faktor VII-Konzentration ist für die beiden hier verwendeten Reagenzien sehr ähnlich.

### Erläuterungen zu den beigefügten Abbildungen

### Fig. 1: pH-Abhängigkeit der Relipidisierung

Die Reagenzien wurden wie in Beispiel 1 und Beispiel 2 beschrieben hergestellt. Phospholipon 25 P wurde mit Glyzinpuffern verschiedener pH-Werte vorgemischt. Nach Zugabe des Gewebsfaktor-Apoproteins wurde der pH gemessen und der Ansatz anschließend neutralisiert. Die Prothrombinzeit PT eines humanen Normalplasmapools wurde koagulometrisch an einem Koagulometer nach Schnitger und Groß bestimmt (Mittelwert einer Doppelbestimmung) .

### Fig. 2: Zeitabhängigkeit der Relipidisierung im sauren

Die Reagenzienherstellung erfolgte wie in Beispiel 1 und Beispiel 3 beschrieben. Dargestellt ist die Prothrombinzeit PT eines humanen Normalplasmapools (Mittelwert einer Doppelbestimmung) in Abhängigkeit von der Länge der Inkubation bei saurem pH.

### Fig. 3: Abhängigkeit der Relipidisierung von der Temperatur des Hitzeschritts

Die Reagenzienherstellung ist in Beispiel 4 beschrieben. Dargestellt ist hier die Prothrombinzeit eines humanen Normalplasmapools (Mittelwert einer Doppelbestimmung) in Abhängigkeit von der Temperatur des fünfminütigen Hitzeschritts.

### Fig. 4: Zeitabhängigkeit der Relipidisierung durch Erhitzen

Dargestellt ist die Prothrombinzeit PT eines humanen Normalplasmapools (Mittelwert einer Doppelbestimmung) in Abhängigkeit von der Länge einer Hitzebehandlung bei 95°C.

### Fig. 5: Faktor VII-Empfindlichkeit

Die Herstellung des Reagenzes erfolgte gemäß Beispiel 1, die Verdünnung des humanen Normalplasmapools mit Faktor VII-Mangelplasma erfolgte gemäß Beispiel 5.

Dargestellt ist die Abhängigkeit der relativen Prothrombinzeit [Ratio (PT)/(PT_{100 % Faktor VII})] in Abhängigkeit von der Faktor VII-Konzentration. Das aus rekombinantem Gewebsfaktor gemäß einem erfindungsgemäßen Verfahren hergestellte Reagenz wurde verglichen mit einem nativen, partiell aufgereinigten Gewebsfaktor aus Humanplazenta (Thromborel S, Behringwerke AG).

## Patentansprüche

1. Verfahren zur Reaktivierung gereinigter Membranproteine dadurch gekennzeichnet, daß die Umsetzung der Membran proteine mit Phospholipiden ohne Zusatz von Detergenzien im Sauren und/oder bei erhöhter Temperatur durchgeführt wird.

2. Verfahren nach Anspruch 1 dadurch gekennzeichnet, daß der pH-Wert zwischen 1 und 5 liegt.

3. Verfahren nach Anspruch 1 dadurch gekennzeichnet, daß mit einer Temperatur zwischen 50°C und 130°C gearbeitet wird.

4. Verfahren nach Anspruch 1 dadurch gekennzeichnet, daß der pH-Wert zwischen pH 1 und 5 liegt und bei einer Temperatur zwischen 50°C und 130°C reaktiviert wird.

5. Verfahren nach mindestens einem der Ansprüche 1 bis 4 dadurch gekennzeichnet, daß mit reinen Phospoholipiden pflanzlichen oder tierischen Ursprungs, definierten Mischungen reiner Phospholipide oder natürlichen Phospholipidmischungen gearbeitet wird.

6. Verfahren nach mindestens einem der Ansprüche 1 bis 5 dadurch gekennzeichnet, daß mit gelösten Membranproteinen gearbeitet wird.

7. Verfahren nach mindestens einem der Ansprüche 1 bis 6 dadurch gekennzeichnet, daß die Reaktivierung bei einem an eine Affinitätssäule gebundenen Membranprotein durchgeführt wird.

8. Verfahren nach mindestens einem der Ansprüche 1 bis 7 dadurch gekennzeichnet, daß eine Immunadsorptionssäule eingesetzt wird.

9. Verfahren nach Anspruch 8 dadurch gekennzeichnet, daß ein oder mehrere monoklonaler Antikörper gegen das Membranprotein eingesetzt worden.

10. Verfahren nach Anspruch 8 dadurch gekennzeichnet, daß polyklonale Antikörper eingesetzt werden.

11. Verfahren nach mindestens einem der Ansprüche 1 bis 10, dadurch gekennzeichnet, daß mit Gewebsfaktor-Apoprotein gearbeitet wird.

12. Verfahren nach Anspruch 11 dadurch gekennzeichnet, daß das Gewebsfaktor-Apoprotein humanen, tierischen oder rekombinanten Ursprungs ist.

13. Verfahren nach Anspruch 11 oder 12 dadurch gekenn zeichnet, daß mit einer Mutante des Gewebsfaktor-Apoproteins gearbeitet wird.

14. Detergenzfrei reaktiviertes Gewebs-Apoprotein, erhält lich nach mindestens einem der Verfahren nach Anspruch 1 bis 13.

15. Reagenz zur Bestimmung der Prothrombinzeit dadurch gekennzeichnet, daß es Gewebsfaktor nach Anspruch 14 enthält.

16. Pharmazeutische Komposition dadurch gekennzeichnet daß sie Gewebsfaktor nach Anspruch 14 enthält.

## Claims

1. A process for reactivating purified membrane proteins, wherein the reaction of the membrane proteins with phospholipids is carried out under acid conditions and/or at elevated temperature without adding detergents.

2. The process as claimed in claim 1, wherein the pH is between 1 and 5.

3. The process as claimed in claim 1, which is carried out at a temperature of between 50°C and 130°C.

4. The process as claimed in claim 1, wherein the pH is between pH 1 and 5 and reactivation takes place at a temperature between 50°C and 130°C.

5. The process as claimed in at least one of claims 1 to 4, which is carried out using pure phospholipids of vegetable or animal origin, defined mixtures of pure phospholipids, or natural phospholipid mixtures.

6. The process as claimed in at least one of claims 1 to 5, which is carried out using dissolved membrane proteins.

7. The process as claimed in at least one of claims 1 to 6, wherein the reactivation is carried out with a membrane protein which is bound to an affinity column.

8. The process as claimed in at least one of claims 1 to 7, wherein an immunoadsorption column is employed.

9. The process as claimed in claim 8, wherein one or more monoclonal antibodies against the membrane protein are employed.

10. The process as claimed in claim 8, wherein polyclonal antibodies are employed.

11. The process as claimed in at least one of claims 1 to 10, which is carried out using tissue factor apoprotein.

12. The process as claimed in claim 11, wherein the tissue factor apoprotein is of human, animal or recombinant origin.

13. The process as claimed in claim 11 or 12, which is carried out using a mutant of tissue factor apoprotein.

14. A detergent-free, reactivated tissue apoprotein, which can be obtained by at least one of the processes as claimed in claims 1 to 13.

15. A reagent for determining the prothrombin time, which reagent contains tissue factor as claimed in claim 14.

16. A pharmaceutical composition, which contains tissue factor as claimed in claim 14.

## Revendications

1. Procédé pour la réactivation de protéines membranaires purifiées, caractérisé en ce que l'on effectue la réaction des protéines membranaires avec des phospholipides, sans addition de détergents, dans des acides et/ou à haute température.

2. Procédé selon la revendication 1, caractérisé en ce que le pH est entre 1 et 5.

3. Procédé selon la revendication 1, caractérisé en ce que l'on opère à une température comprise entre 50 et 130°C.

4. Procédé selon la revendication 1, caractérisé en ce que le pH est entre 1 et 5 et la réactivation est effectuée à une température comprise entre 50 et 130°C.

5. Procédé selon au moins l'une des revendications 1 à 4, caractérisé en ce que l'on opère avec des phospholipides purs d'origine animale ou végétale, des mélanges définis de phospholipides purs ou des mélanges de phospholipides naturels.

6. Procédé selon au moins l'une des revendications 1 à 5, caractérisé en ce que l'on opère avec des protéines membranaires dissoutes.

7. Procédé selon au moins l'une des revendications 1 à 6, caractérisé en ce que la réactivation est effectuée sur une protéine membranaire fixée à une colonne d'affinité.

8. Procédé selon au moins l'une des revendications 1 à 7, caractérisé en ce que l'on utilise une colonne d'immuno-adsorption.

9. Procédé selon la revendication 8, caractérisé en ce que l'on utilise un ou plusieurs anticorps monoclonaux dirigés contre la protéine membranaire.

10. Procédé selon la revendication 8, caractérisé en ce que l'on utilise des anticorps polyclonaux.

11. Procédé selon au moins l'une des revendications 1 à 10, caractérisé en ce que l'on opère avec une apoprotéine de facteur tissulaire.

12. Procédé selon la revendication 11, caractérisé en ce que l'apoprotéine de facteur tissulaire est d'origine humaine, animale ou recombinante.

13. Procédé selon la revendication 11 ou 12, caractérisé en ce que l'on opère avec un mutant de l'apoprotéine de facteur tissulaire.

14. Apoprotéine tissulaire réactivée sans détergent, pouvant être obtenue selon au moins l'un des procédés conformes aux revendications 1 à 13.

15. Réactif pour la détermination du temps de prothrombine, caractérisé en ce qu'il contient un facteur tissulaire selon la revendication 14.

16. Composition pharmaceutique, caractérisée en ce qu'elle contient un facteur tissulaire selon la revendication 14.
